Europäisches Patentamt
European Patent Office
Office européen des brevets

(11) Veröffentlichungsnummer: **0 345 689**
**A2**

# EUROPÄISCHE PATENTANMELDUNG

(21) Anmeldenummer: 89110131.3

(22) Anmeldetag: 05.06.89

(51) Int. Cl.[4]: C07C 29/56 , C07C 35/06 , C07C 35/08

(30) Priorität: 06.06.88 CH 2148/88

(43) Veröffentlichungstag der Anmeldung: 13.12.89 Patentblatt 89/50

(84) Benannte Vertragsstaaten: CH DE FR GB IT LI NL

(71) Anmelder: CIBA-GEIGY AG
Klybeckstrasse 141
CH-4002 Basel(CH)

(72) Erfinder: Scheffold, Rolf, Prof. Dr.
Multengutstrasse 31
CH-3074 Muri b. Bern(CH)
Erfinder: Walder, Lorenz, Dr.
Rue du Bassin 14
CH-2000 Neuchâtel(CH)

(74) Vertreter: Zumstein, Fritz jun., Dr. et al
Dr. F. Zumstein sen. Dr. E. Assmann Dr. R. Koenigsberger Dipl.-Ing. F. Klingseisen Dr. F. Zumstein jun. Bräuhausstrasse 4
D-8000 München 2(DE)

## (54) Verfahren zur Herstellung von alpha,beta-ungesättigten Alkoholen.

(57) Cyclische Epoxide oder offenkettige mit einer nicht-terminalen Epoxygruppe Epoxide werden unter der Einwirkung eines Kobalt(I)-Derivats von Vitamin $B_{12}$ zu $\alpha,\beta$-ungesättigten sekundären Alkoholen isomerisiert. Prochirale Epoxide ergeben dabei optisch aktive Allylalkohole. Die Alkohole eignen sich für die asymmetrische Oxydation von ungesättigten Alkoholen zu Epoxiden, die wertvolle Zwischenprodukte für Synthesen sind.

EP 0 345 689 A2

## Verfahren zur Herstellung von α,β-ungesättigten Alkoholen

Die Erfindung betrifft ein Verfahren zur Isomerisierung von Epoxiden zu α,β-ungesättigten Alkoholen in Gegenwart katalytischer Mengen eines Kobalt(I)-Derivats von Vitamin $B_{12}$ oder eines Vitamin $B_{12}$-Derivates.

A. Fischli beschreibt in Helvetica Chimica Acta, Vol. 65, S. 1167-1190 (1982) die Bildung eines Produktegemisches bei der Umsetzung von 10,11-Epoxyundecylacetat unter reduktiven Bedingungen (Zink Eisessig) und in Gegenwart katalytischer Mengen Kob(I)alamin oder Heptamethylkob(I)yrinat. Es wird ausdrücklich darauf hingewiesen, dass keine Bildung von α,β-ungesättigten Alkoholen beobachtet wird.

Es wurde gefunden, dass Epoxide in Gegenwart katalytischer Mengen von Kobalt(I)-Derivaten des Vitamins $B_{12}$ oder von Vitamine $B_{12}$-Derivaten dann in hohen Ausbeuten zu α,β-ungesättigten sekundären Alkohlen isomerisiert werden, wenn man Epoxide mit einer nicht-terminalen Epoxygruppe verwendet.

Ein Gegenstand vorliegender Erfindung ist ein Verfahren zur Herstellung von Verbindungen der Formel I

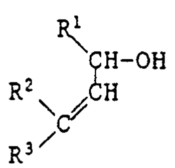

(I),

worin $R^2$ und $R^3$ unabhängig voneinander H, und $R^1$, $R^2$ und $R^3$ unabhängig voneinander lineares $C_1$-$C_{18}$-Alkyl oder $C_2$-$C_{18}$-Alkenyl, $C_3$-$C_8$-Cycloalkyl, $C_3$-$C_8$-Cycloalkenyl, $C_6$-$C_{10}$-Aryl, $C_4$-$C_8$-Cycloheteroalkyl mit O oder S als Heteroatome, $C_4$-$C_8$-Cycloheteroalkenyl mit -O-, -S- oder -N= als Heteroatome oder $C_4$-$C_9$-Heteroaryl mit -O-, -S- oder =N- als Heteroatome, bedeuten, oder $R^1$ und $R^2$ zusammen lineares $C_1$-$C_9$-Alkylen oder $C_2$-$C_9$- Alkenylen, in α,β-Stellung gebundenes $C_6$-$C_{10}$-Arylen oder $C_4$-$C_9$-Heteroarylen mit -O-, -S-oder =N- als Heteroatome oder in α,β-Stellung gebundenes $C_7$-$C_{10}$-Arylmethylen oder $C_4$-$C_9$-Heteroarylmethylen mit -O-, -S-oder =N- als Heteroatome darstellen, wobei $R^1$, $R^2$ und $R^3$ unsubstituiert oder mit -OH, -CN, =O, Halogen, $C_1$-$C_{12}$-Alkyl, $R^4$-X- mit X gleich -O-, -S-, -SO-, -$SO_2$-, -CO- und $R^4$ gleich $C_1$-$C_{12}$-Alkyl, Benzyl oder Phenyl, -$COOR^5$ mit $R^5$ gleich H, $C_1$-$C_{12}$-Alkyl, Phenyl oder Benzyl oder -$CONR^5R^6$ mit $R^6$ unabhängig in der gleichen Bedeutung von $R^5$, $R^7$-CO-O- oder $R^7$-CO-$NR^5$- mit $R^7$ gleich $C_1$-$C_6$-Alkyl-, $C_2$-$C_6$-Alkenyl oder $C_1$-$C_6$-Halogenalkyl, $C_5$- oder $C_6$-Cycloalkyl, Phenyl oder Halogenphenyl oder Benzyl, substituiert ist, dadurch gekennzeichnet, dass man in einem inerten polaren Lösungsmittel bei einer Temperatur von -20 °C bis +60 °C katalytische Mengen eines Kobalt(I)-Derivats von Vitamin $B_{12}$ oder eines Vitamin-$B_{12}$-Derivats auf ein Epoxid der Formel II

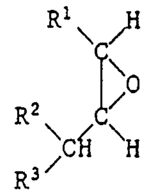

(II)

einwirken lässt, worin $R^1$, $R^2$ und $R^3$ die zuvor angegebenen Bedeutungen haben.

$R^1$, $R^2$ und $R^3$ enthalten in der Bedeutung von linearem Alkyl bevorzugt 1 bis 12, besonders 1 bis 6 C-Atome. Die Substitution mit $C_1$-$C_{12}$-Alkyl umfasst verzweigtes Alkyl. Beispiele sind Methyl, Ethyl und die Isomeren von Propyl, Butyl, Pentyl, Hexyl, Heptyl, Octyl, Nonyl, Decyl, Undecyl, Dodecyl, Tetradecyl, Hexadecyl und Octadecyl.

$R^1$, $R^2$ und $R^3$ enthalten in der Bedeutung von Alkenyl bevorzugt 2 bis 12, besonders 2 bis 6 C-Atome. Die Substitution mit Alkyl umfasst verzweigtes Alkenyl. Einige Beispiele sind Ethenyl, Propen-1- oder -2-yl, But-1-en-1-yl, But-2-en-1-yl, But-1-en-2-yl, But-1-en-4-yl, Pent-1-en-1-yl oder -2-yl oder -5-yl, Hexenyl, Heptenyl, Octenyl, Decenyl, Dodecenyl, Hexadecenyl und Octadecenyl.

$R^1$, $R^2$ und $R^3$ enthalten in der Bedeutung von Cycloalkyl und Cycloalkenyl bevorzugt 5 bis 8, besonders 5 oder 6 C-Atome. Beispiele sind Cyclopropyl, Cyclobutyl, Cyclopentyl, Cyclohexyl, Cycloheptyl, Cyclooctyl, Cycloprop-1-en-1-yl oder -3-yl, Cyclobut-1-en-1- oder -3-yl, Cyclopent-1-en-1- oder -3- oder -4-yl, Cyclohex-1-en-1- oder -3- oder -4-yl, Cycloheptenyl und Cyclooctenyl.

$R^1$, $R^2$ und $R^3$ als Aryl stellen insbesondere Naphthyl oder Phenyl dar.

$R^1$, $R^2$ und $R^3$ in der Bedeutung von Cycloheteroalkyl und Cycloheteroalkenyl enthalten bevorzugt 4 oder 5 C-Atome und bevorzugt 1 oder 2 Heteroatome sowie bevorzugt 5 oder 6 Ringatome. Einige Beispiele sind Tetrahydrofuranyl, Tetrahydrothiophenyl, Tetrahydropyranyl, Tetrahydrothiapyranyl, Oxepanyl, Thiepanyl, Azepanyl, Dihydrofuranyl, Dihydrothiophenyl, 3H,4H-Pyrryl, Dihydropyranyl, Dihydrothiapyranyl, 3H, 5H-Pyridinyl, Pyranyl, Thiapyranyl.

$R^1$, $R^2$ und $R^3$ enthalten in der Bedeutung von Heteroaryl bevorzugt 4 oder 5 C-Atome und vorzugsweise 5 oder 6 Ringatome sowie vorzugsweise 1 oder 2 Heteroatome. Einige Beispiele sind Furanyl, Thiophenyl, Pyridyl, Chinolinyl, Pyridazinyl, Pyrimidinyl, Pyrazinyl, Benzofuranyl, Benzoxazolyl, Chinazolinyl, Chinoxalinyl.

$R^1$ und $R^2$ zusammen enthalten in der Bedeutung von Alkylen und Alkenylen bevorzugt 2 bis 6, besonders 2 bis 4 C-Atome. Die Substitution mit Alkyl umfasst verzweigtes Alkylen und Alkenylen. Einige Beispiele sind Methylen, 1,1- oder 1,2-Ethylen, 1,1-, 1,2- oder 1,3-Propylen, 1,1-, 2,2-, 1,2-, 1,3- oder 1,4-Butylen, 1,2-, 1,3-, 1,4- oder 1,5-Pentylen, Hexylen, Heptylen, Octylen, Nonylen, Ethenylen, 1,2- oder 1,3-Propenylen, 1,2-, 1,3- oder 1,4-But-1- oder -2-enyl, Pentenyl und Hexenyl.

$R^1$ und $R^2$ zusammen in der Bedeutung von Arylen sind z.B. 1,2- oder 2,3-Naphthylen und 1,2-Phenylen.

$R^1$ und $R^2$ als Heteroarylen sind z.B. 1,2- oder 2,3-Furanylen, 1,2- oder 2,3-Thiophenylen, 2,3- oder 3,4-Pyridylen und 2,3-Pyrimidinylen.

$R^1$ und $R^2$ in der Bedeutung von Arylmethylen und Heteroarylmethylen können z.B. Phen-1-yl-2-methylen, Furan-2-yl-3-methylen, Pyrid-3-yl-4-methylen oder Pyrid-4-yl-3-methylen sein.

$R^1$, $R^2$ und $R^3$ können ein- oder mehrfach, vorzugsweise ein- bis fünffach, besonders ein- bis dreifach und insbesondere ein- oder zweifach substituiert sein. Halogen ist vorzugsweise -F oder -Cl. Der Substituent Alkyl enthält bevorzugt 1 bis 6 C-Atome und kann linear oder verzweigt sein. $R^4$, $R^5$ und $R^6$ sind vorzugsweise $C_1$-$C_6$-Alkyl, Phenyl oder Benzyl, wobei $R^5$ und $R^6$ auch H bedeuten können. $R^7$ ist als Halogenalkyl vorzugsweise Fluor- oder Chloralkyl und als Halogenphenyl Fluor- oder Chlorphenyl.

In einer bevorzugten Ausführungsform steht $R^3$ für H und in einer anderen bevorzugten Ausführungsform stellt $R^1$ einen der definierten Reste dar. Eine Ausführungsform ist dadurch gekennzeichnet, dass in Formel I $R^2$ und $R^3$ für H, und $R^1$, $R^2$ oder $R^3$ unsubstituiertes oder substituiertes $C_1$-$C_{12}$-Alkyl oder $C_2$-$C_{12}$-Alkenyl, $C_5$-$C_8$-Cycloalkyl oder -Cycloalkenyl, $C_6$-$C_{10}$-Aryl oder $C_4$-$C_9$-Heteroaryl mit -O-, -S- oder =N- als Heteroatome, stehen, oder $R^1$ und $R^2$ zusammen unsubstituiertes oder substituiertes $C_2$-$C_4$-Alkylen oder -Alkenylen, $C_6$-$C_{10}$-Aryl-1,2-en, $C_4$- oder $C_5$-Heteroaryl-1,2-en, $C_6$-$C_{10}$-Ar-1-yl-2-methylen oder $C_4$- oder $C_5$-Heteroar-1-yl-2-methylen mit -O-, -S-oder =N- als Heteroatome darstellen.

In einer vorteilhaften Ausführungsform bedeuten $R^2$ H, und $R^1$ oder $R^2$ substituiertes oder unsubstituiertes $C_1$-$C_6$-Alkyl, $C_2$-$C_6$-Alkenyl, Phenyl oder Pyridyl. $R^3$ bedeutet als Rest vorzugsweise substituiertes oder unsubstituiertes $C_1$-$C_6$-Alkyl, $C_2$-$C_6$-Alkenyl, Phenyl oder Pyridyl.

$R^1$ und $R^2$ zusammen bedeuten bevorzugt unsubstituiertes oder substituiertes $C_2$-$C_4$-Alkylen, $C_2$-$C_4$-Alkenylen, oder unsubstituiertes oder substituiertes, in $\alpha,\beta$-Stellung gebundenes Phenylen, Pyridylen, Benzylen oder Pyridylmethylen.

Es wurde ferner gefunden, dass bei der Umsetzung ein Ueberschuss eines Enantiomeren aus einem prochiralen Epoxid der Formel II gebildet werden kann. Besonders vorteilhaft ist hierbei die Verwendung der Mesoform, da hierbei ein Ueberschuss nur eines Enantiomeren beobachtet wird. Eine besonders bevorzugte Ausführungsform des Verfahrens ist daher dadurch gekennzeichnet, dass man als Epoxid der Formel II ein prochirales Epoxid in Mesokonfiguration verwendet, und Verbindungen der Formel I herstellt, in denen Enantiomere der Formel Ia

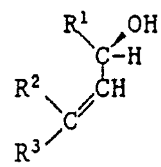

(Ia),

im Ueberschuss enthalten sind. Der Enantiomerenüberschuss beträgt bevorzugt mindestens 30 % und kann 50 % und mehr betragen.

Geeignete Lösungsmittel sind z.B. polare protische Lösungsmittel wie z.B. Wasser und Alkohole; oder Ether sowie deren Gemische mit Wasser. Einige Beispiele sind Methanol, Ethanol, Propanol, Butanol, Ethylenglykol, Diethylenglykol, Ethylenglykolmonomethylether, Diethylenglykolmonomethylether, Ethylenglykoldimethyl- oder -diethylether, Diethylenglykoldimethylether, Tetrahydrofuran und Dioxan.

Die Reaktionstemperatur beträgt bevorzugt -10 °C bis +50 °C und besonders bevorzugt wird das Verfahren bei Raumtemperatur durchgeführt. Die Reaktion kann durch die Einwirkung von Licht beschleunigt werden.

Die Menge des Katalysators, also des Kobalt(I)-Derivats von Vitamin $B_{12}$ oder eines Derivats davon beträgt bevorzugt 0,01 bis 10 Mol-%, besonders 0,1 bis 5 Mol-%, bezogen auf das Epoxid der Formel II.

Das Verfahren wird zweckmässig unter Inertgasatmosphäre durchgeführt, wobei Stickstoff oder Edelgase verwendet werden können. Kobalt(I)-Derivate von Vitamin-$B_{12}$ und seinen Derivaten sowie deren Herstellung durch Reduktion von Vitamin $B_{12}$ und seinen Derivaten mit Metallen wie z.B. Zink, oder $NaBH_4$, $LiAlH_4$ oder elektrochemischen Methoden sind an sich bekannt, siehe z.B. Helvetica Chimica Acta, Vol. 65, S. 1167-1190 (1982) und Z. Schneider und A. Stroinski, Comprehensive $B_{12}$, Verlag W. de Gruyter, Berlin, New York (1987).

Als Derivate von Vitamin $B_{12}$ kommen z.B.

solche in Frage, worin die Axialliganden durch andere Liganden ersetzt sind oder die Seitenketten derivatisiert sind. Geeignet sind auch Kobyrinate, deren Estergruppen vorzugsweise $C_1$-$C_6$-Alkylgruppen enthalten. Besonders bevorzugt werden im erfindungsgemässen Verfahren Kob(I)alamin oder Heptamethylkob(I)yrinat verwendet.

Epoxide der Formel II sind bekannt, käuflich erhältlich oder durch die Epoxidierung von entsprechenden Alkenen leicht herstellbar.

Das Verfahren kann z.B. so ausgeführt werden, dass man zunächst Vitamin $B_{12}$ oder ein Derivat reduziert. Im allgemeinen wird ein Ueberschuss an Reduktionsmittel verwendet, der zum Schutz gegen eine Oxidation im Reaktionsgemisch verbleiben kann. Die vollständige Reduktion ist am Farbumschlag der Lösung von rot nach grün erkennbar. Nach der Reduktion wird das Epoxid zugegeben und man lässt noch einige Zeit rühren.

Die Isolierung der Verbindungen der Formel I kann nach üblichen Methoden erfolgen, z.B. durch Extraktion, Destillation oder Kristallisation. Das erhaltene Produkt kann durch Anwendung chromatographischer Methoden weiter gereinigt werden.

Mit dem erfindungsgemässen Verfahren können leicht zugängliche Epoxide in hohen Ausbeuten unter milden Reaktionsbedingungen zu $\alpha,\beta$-ungesättigten Alkoholen isomerisiert werden, die wertvolle Zwischenprodukte für organische Synthesen darstellen. Besonders vorteilhaft ist, dass auch die Herstellung von reinen Enantiomeren durch deren Isolierung aus Verbindungen der Formel I mit einem Enantiomerenüberschuss möglich ist. Der ökologisch unbedenkliche Katalysator kann wiederholt eingesetzt werden.

Ein weiterer Gegenstand der Erfindung ist die Verwendung von Kobalt(I)-Derivaten des Vitamin $B_{12}$ oder Derivaten des Vitamin $B_{12}$ als Katalysator zur Isomerisierung von cyclischen Epoxiden oder offenkettigen Epoxiden zu $\alpha,\beta$-ungesättigten Alkoholen.

Die Verbindungen der Formel I können z.B. in der Synthese auf den Gebieten der Alkaloide, Steroide oder Prostaglandine [vgl. G. Stork, Bull. Chem. Soc. Jpn., 61, S. 149-154 (1988)], zur sigmatropen Umlagerung vom Typus der Claisenumlagerung und verwandter Umlagerungen zum Aufbau von C-Gerüsten von Feinchemikalien [vgl. R.K. Hill, Asymmetric Synthesis, Ed. J.D. Morrison, Vol. 3/Part B, Academic Press, S. 511 bis 565 (1984)] oder zur asymmetrischen Epoxidierung nach Sharpless (vgl. EP-A-0 046 033) verwendet werden.

Die nachfolgenden Beispiele erläutern die Erfindung näher.

Beispiel 1:

a) Zinkaktivierung:

Zinkwolle (0,8 g, 12 mMol) wird um einen Teflon-überzogenen Magnetrührer gewickelt und in 100 ml 1 N HCl während 15 min gerührt. Anschliessend wird mit 5 mal 20 ml Wasser und 5 mal 20 ml Methanol gewaschen.

b) Herstellung von (1R)-Cyclohex-2-en-1-ol:

Zu 40 ml Methanol in einem Zweihalskolben mit Argon-Schutzgasüberleitung wird Hydroxycobalaminhydrochlorid (0,42 g, 0,3 mmol) und der mit frisch aktivierter Zinkwolle (0,82 g, 12 mmol) umwickelte Magnetrührer gegeben. Die Reduktion von Vitamin $B_{12a}$ zu Vitamin $B_{12s}$ ist in 1 Stunde vollständig abgelaufen (Farbänderung rot nach grün). Zu der auf 40 °C thermostatisierten Lösung wird Cyclohexenoxyd (2,97 g, 30 mmol) gegeben (Farbänderung grün nach braun). Nachdem sich die Lösung wiederum grün verfärbt hat (nach ca. 40 Stunden), wird das Zink entfernt, mit Methanol gewaschen und die vereinigten methanolischen Lösungen am Rotationsverdampfer aufkonzentriert. Zum Rückstand werden 10 ml Diethylether gegeben, wobei der Katalysator und gebildete anorganische Salze ausfallen. Die etherische Phase wird abgetrennt und die Feststoffe mit 5 mal 2 ml Diethylether gewaschen. Die vereinigten etherischen Phasen werden durch Silicagel (1 g) filtriert und eingeengt (2,95 g gelbliches Oel). Nach einer Kugelrohrdestillation des Rohproduktes erhält man 2,07 g einer farblosen Flüssigkeit, die zu 78,8 % Cyclohexenol enthält (chemische Ausbeute 56 %). Im Produkt ist das R-Isomere (1) mit einem Enantiomerenüberschuss von 38 % angereichert (Vergleich der optischen Drehung des Rohproduktes mit dem Literaturwert von 1, $[\alpha]_D$ = 112°, S. Yamada, N Takamura, T. Mizoguchi, Chem. Pharm. Bull, 23, 2539 (1975) unter Berücksichtigung der Reinheit berechnet). Das zu 78,8 % reine Cyclohexenol wird direkt analysiert.

Physikalische Daten:

$^1$H-NMR: $\delta$ ($CDCl_3$, 60 MHz): 1,50-2,50 (m,7H), 4,32 (m,1H), 5,90 (m,2H). Optische Drehung (C=1,736 in $CHCl_3$, 1=1 dm, $\lambda$=589: $[\alpha]_D^{20}$ = +33,87°.

Beispiel 2: Herstellung von (1R/-Cyclopent-2-en-1-ol:

In 15 ml Methanol in einem 100 ml Zweihalskolben mit Argon-Schutzgasüberleitung wird Hydroxykobalaminhydrochlorid (0,84 g, 0,6 mmol) gelöst, im Eisbad auf 0 °C gekühlt und Natriumborhydrid (0,113 g, 3,0 mmol) in zwei Portionen zugegeben. Die Farbe verändert sich innerhalb von kurzer Zeit von rot nach dünkelgrün. Dann wird mit einer Spritze Cyclopentenoxid (1,68 g, 20 mmol) zugegeben (Farbänderung dunkelgrün nach braun) und die Lösung 48 Stunden bei Raumteperatur (22 °C) mit einem Magnetrührer gerührt. Dann wird die Apparatur geöffnet und 80 ml Diethylether zugegeben, wobei sich ein dunkelbrauner Niederschlag bildet, welcher rasch sedimentiert. Die überstehende farblose Lösung wird abdekantiert und der Niederschlag fünf mal mit je 30 ml Diethylether ausgezogen. Die vereinigten Etherextrakte werden am Rotationsverdampfer eingeengt, wobei 1,56 g eines farblosen Oels verbleiben. Gemäss NMR und GC handelt es sich dabei um (1R)-Cyclopent-2-en-1-ol (Reinheit 94 %), Ausbeute: 87 %.

Physikalische Daten:

$^1$H-NMR: $\delta$ ($CDCl_3$, 60 MHz): 1,45-2,60 (m,5H), 4,9 (m,1H), 5,92 (m,2H). Optische Drehung (C = 1.1 in $CCl_4$, 1 = 1 dm, $\lambda$ = 578: $[\alpha]_D^{22}$ = +75,4°

Der Enantiomerenüberschuss beträgt ca. 55 % (gemäss $^1$H-NMR der entsprechenden Mosher-Ester).

## Ansprüche

1. Verfahren zur Herstellung von Verbindungen der Formel I

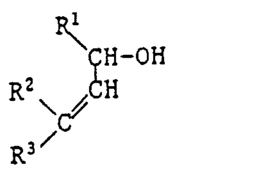

(I),

worin $R^2$ und $R^3$ unabhängig voneinander H, und $R^1$, $R^2$ und $R^3$ unabhängig voneinander lineares $C_1$-$C_{18}$-Alkyl oder $C_2$-$C_{18}$-Alkenyl, $C_3$-$C_8$-Cycloalkyl, $C_3$-$C_8$-Cycloalkenyl, $C_6$-$C_{10}$-Aryl, $C_4$-$C_8$-Cycloheteroalkyl mit 0 oder S als Heteroatome, $C_4$-$C_8$-Cycloheteroalkenyl mit -O-, -S- oder -N= als Heteroatome, oder $C_4$-$C_9$-Heteroaryl mit -O-, -S- oder =N- als Heteroatome bedeuten, oder $R^1$ und $R^2$ zusammen lineares $C_1$-$C_9$-Alkylen oder $C_2$-$C_9$-Alkenylen, in $\alpha,\beta$-Stellung gebundenes $C_6$-$C_{10}$-Arylen oder $C_4$-$C_9$-Heteroarylen mit -O-, -S-oder =N- als Heteroatomen oder in $\alpha,\beta$-Stellung gebundenes $C_7$-$C_{10}$-Arylmethylen oder $C_4$-$C_9$-Heteroarylmethylen mit -O-, -S-oder =N- als Heteroatome darstellen, wobei $R^1$, $R^2$ und $R^3$ unsubstituiert oder mit -OH, -CN, =O, Halogen, $C_1$-$C_{12}$-Alkyl, $R^4$-X- mit X gleich -O-, -S-, -SO-, $-SO_2$, -CO- und $R^4$ gleich $C_1$-$C_{12}$-Alkyl, Benzyl oder Phenyl, $-COOR^5$ mit $R^5$ gleich H, $C_1$-$C_{12}$-Alkyl, Phenyl oder Benzyl oder $-CONR^5R^6$ mit $R^6$ unabhängig in der gleichen Bedeutung von $R^5$, $R^7$-CO-O- oder $R^7$-CO-$NR^5$- mit $R^7$ gleich $C_1$-$C_6$-Alkyl-, $C_2$-$C_6$-Alkenyl oder $C_1$-$C_6$-Halogenalkyl, $C_5$- oder $C_6$-Cycloalkyl, Phenyl oder Halogenphenyl oder Benzyl, substituiert ist, dadurch gekennzeichnet, dass man in einem inerten polaren Lösungsmittel bei einer Temperatur von -20 °C bis +60 °C katalytische Mengen eines Kobalt(I)-Derivats von Vitamin $B_{12}$ oder eines Vitamin-$B_{12}$-Derivats auf ein Epoxid der Formel II

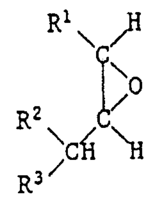

(II)

einwirken lässt, worin $R^1$, $R^2$ und $R^3$ die zuvor angegebenen Bedeutungen haben.

2. Verfahren gemäss Anspruch 1, dadurch gekennzeichnet, dass in Formel I $R^2$ und $R^3$ für H, und $R^1$, $R^2$ und $R^3$ unsubstituiertes oder substituiertes $C_1$-$C_{12}$-Alkyl oder $C_2$-$C_{12}$-Alkenyl, $C_5$-$C_8$-Cycloalkyl oder -Cycloalkenyl, $C_6$-$C_{10}$-Aryl oder $C_4$-$C_9$-Heteroaryl mit -O-, -S- oder =N- als Heteroatome, stehen, oder $R^1$ und $R^2$ zusammen unsubstituiertes oder substituiertes $C_2$-$C_4$-Alkylen oder -Alkenylen, $C_6$-$C_{10}$-Aryl-1,2-en, $C_4$- oder $C_5$-Heteroaryl-1,2-en mit -O-, -S- oder -N= als Heteroatome, $C_6$-$C_{10}$-Ar-1-yl-2-methylen oder $C_4$- oder $C_5$-Heteroar-1-yl-2-methylen mit -O-, -S- oder =N-als Heteroatome darstellen.

3. Verfahren gemäss Anspruch 1, dadurch gekennzeichnet, dass $R^3$ in Formel I für H steht.

4. Verfahren gemäss Anspruch 1, dadurch gekennzeichnet, dass $R^1$ substituiertes oder unsubstituiertes $C_1$-$C_6$-Alkyl, $C_2$-$C_6$-Alkenyl, Phenyl oder Pyridyl steht.

5. Verfahren gemäss Anspruch 1, dadurch gekennzeichnet, dass $R^2$ für H oder unsubstituiertes oder substituiertes $C_1$-$C_6$-Alkyl, $C_2$-$C_6$-Alkenyl, Phenyl oder Pyridyl steht.

6. Verfahren gemäss Anspruch 1, dadurch gekennzeichnet, dass $R^1$ und $R^2$ zusammen unsubstituiertes oder substituiertes $C_2$-$C_4$-Alkylen, $C_2$-$C_4$-Alkenylen, oder unsubstituiertes oder substituiertes, in $\alpha,\beta$-Stellung gebundenes Phenylen, Pyridylen, Benzylen oder Pyridylmethylen darstellen.

7. Verfahren gemäss Anspruch 1, dadurch gekennzeichnet, dass man als Epoxid der Formel II ein prochirales Epoxid in Mesokonfiguration verwendet, und Verbindungen der Formel I herstellt, in denen Enantiomere der Formel Ia

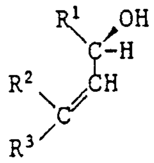

(Ia),

im Ueberschuss enthalten sind.

8. Verfahren gemäss Anspruch 7, worin der Enantiomerenüberschuss mindestens 30 % beträgt.

9. Verfahren gemäss Anspruch 1, dadurch gekennzeichnet, dass das Lösungsmittel Wasser, ein Alkohol, ein Ether oder deren Gemische mit Wasser ist.

10. Verfahren gemäss Anspruch 1, dadurch gekennzeichnet, dass die Temperatur -10°C bis 50°C beträgt.

11. Verfahren gemäss Anspruch 10, dadurch gekennzeichnet, dass es bei Raumtemperatur durchgeführt wird.

12. Verfahren gemäss Anspruch 1, dadurch gekennzeichnet, dass die Menge des Kobalt(I)-Derivats von Vitamin $B_{12}$ oder eines Derivates davon 0,01 bis 10 Mol-% beträgt, bezogen auf das Epoxid der Formel II.

13. Verfahren gemäss Anspruch 1, dadurch gekennzeichnet, dass es sich beim Kobalt(I)-Derivat von Vitamin $B_{12}$ um Kob(I)alamin oder Heptamethylkob(I)yrinat handelt.

14. Verwendung von Kobalt(I)-Derivaten des Vitamin $B_{12}$ oder eines Derivats des Vitamin $B_{12}$ als Katalysator zur Isomerisierung von cyclischen Epoxiden oder offenkettigen mit einer nicht-terminalen Epxoygruppe Epoxiden zu $\alpha,\beta$-ungesättigten sekundären Alkoholen.